# EUROPÄISCHE PATENTSCHRIFT

(11) **EP 1 141 367 B1**
(45) Veröffentlichungstag und Bekanntmachung des Hinweises auf die Patenterteilung: **07.11.2007**
(21) Anmeldenummer: 99966852.8
(22) Anmeldetag: 13.12.1999
(51) Int. Cl.: C12N 15/87

(54) **SYNTHETISCHES NUKLEINSÄURE-PARTIKEL**
SYNTHETIC NUCLEIC ACID PARTICLE
PARTICULE D'ACIDE NUCLEIQUE SYNTHETIQUE

(30) Priorität: 16.12.1998 DE 19858005
(43) Veröffentlichungstag der Anmeldung: 10.10.2001
(73) Patentinhaber: Zimmer, Andreas, 8010 Graz (AT)
(72) Erfinder: JUNGHANS, Monika, D-76829 Landau/Pfalz (DE); ZIMMER, Andreas, D-60437 Frankfurt (DE); KREUTER, Jörg, D-61350 Bad Homburg (DE)
(74) Vertreter: Gassner, Wolfgang
(86) Internationale Anmeldenummer: PCT/DE1999/003974
(87) Internationale Veröffentlichungsnummer: WO 2000/036131

(56) Entgegenhaltungen:
- EP-A- 0 727 223
- WO-A-93/04701
- WO-A-95/25809
- WO-A-98/06437
- LI, S. UND HUANG, L.: "In vivo gene transfer via intravenous administration of cationic lipid-protamine-DNS (LPD) complexes" GENE THERAPY, Bd. 4, 1997, Seiten 891-900, XP000886074 in der Anmeldung erwähnt
- WOLFERT, M.A. UND SEYMOUR, L.W.: "Atomic force microsopic analysis of the influence of the molecular weight of poly(L)lysine on the size of polyelectrolyte complexes formed with DNA" GENE THERAPY, Bd. 3, 1996, Seiten 269-73, XP000886345
- WIENHUES, U. ET AL.: "A novel method for transfection and expression of reconstituted DNA-protein complexes in eukaryotic cells " DNA, Bd. 6, 1987, Seiten 81-9, XP000560129

## Beschreibung

Die Erfindung betrifft ein synthetisches Nucleinsäure-Partikel bzw. Partikel, ein Verfahren zu dessen Herstellung sowie eine Verwendung.

Es ist bekannt, daß Mononukleotide an Clupeine binden (G. D'Auria, L. Paolillo, R. Sartorio and S. Wurzburger (1993): Structure and function of protamines: an 1H nuclear magnetic resonance investigation of the interaction of clupeines with mononucleotides, Biochim. Biophys. Acta, 1162, 209-216).

Nach dem Stand der Technik ist es außerdem bekannt, Komplexverbindungen zwischen doppelsträngigen Oligonukleotiden, polykationischen Polymeren und Lipiden herzustellen. Dazu wird auf die folgenden Literaturstellen verwiesen:
A.V. Kabanov and V.A. Kabanov (1995): DNA Complexes with polycations for the Delivery of Genetic Material into Cells, Bioconjugate Chem., 6, 7-20;
Gao and L. Huang (1996): Potentiation of Cationic Liposome-Mediated Gene Delivery by Polycations, Biochemistry, 35, 1027, 1036;
L. Sorgi, S. Bhattacharya and L. Huang (1997): Protamine sulfate enhances lipid-mediated gene transfer, Gene Therapy, 4, 961-968;
Li and L. Huang (1997): In vivo gene transfer via intravenous administration of cationic lipid-protamine-DNA (LPD) complexes, Gene Therapy, 4, 891-900.

Solche Komplexverbindungen können z.B. zur Transfektion von Plasmid-DNA verwendet werden. Sofern als Polykation an Transferrin gebundenes Protamin verwendet wird, bezeichnet man solche Komplexverbindungen auch als Transferrin-Protamin-DNA-Komplexe. Derartige Komplexe bilden keine kondensierten DNA-Strukturen bzw. Partikel (Wagner, M. Zenke, M. Cotten, H. Beug and M.L. Birnstiel (1990): Transferrin-polycation conjugates as carriers for DNA uptake into cells, Proc. Natl. Acad. Sci. U.S.A., 87, 3410-3414).

Die bekannten Komplexverbindungen können nur aus zuvor gebildeten Partikeln oder bestehenden Komplexen gebildet werden. Dazu muß neben einem Protein auch ein Lipid vorhanden sein. Diese Komplexverbindungen können nachteiligerweise keine partikulären Strukturen aus Oligonukleotiden bilden. Die DNA liegt in der Komplexverbindung nur oberflächlich adsorptiv gebunden vor. Sie kann nachteiligerweise enzymatisch abgebaut werden. Schließlich eignen sich die bekannten Komplexverbindungen nicht zur Herstellung von Arzneimitteln mit Depotwirkung.

Aufgabe der Erfindung ist es, die Nachteile nach dem Stand der Technik zu beseitigen. Es sollen insbesondere ein stabiles synthetisches Partikel und ein Verfahren seiner Herstellung angegeben werden, das eine hohe Transfektionseffektivität ermöglicht. Das synthetische Partikel soll möglichst auch zur Herstellung von Arzneimitteln mit Depotwirkung geeignet sein.

Diese Aufgabe wird durch die Merkmale der Ansprüche 1, 9 und 20 gelöst. Zweckmäßige Ausgestaltungen ergeben sich aus den Merkmalen der Ansprüche 2 bis 8, 10 bis 11 und 21 bis 22.

Nach Maßgabe der Erfindung besteht ein synthetisches Partikel aus einem Oligonukleotid oder einem Derivat davon und einem Protamin, einer Protaminbase, einem Protaminderivat oder - salz mit einem Molekulargewicht im Bereich von 3900 bis 4300, wobei das Gewichtsverhältnis zwischen dem Protamin und Oligonukleotid zwischen 1:1 und 5:1 beträgt. Ein solches synthetisches Partikel ist insbesondere gegenüber einem enzymatischen Abbau stabil. Es ermöglicht eine hohe Transfektionseffizienz sowie die Herstellung von Arzneimitteln mit Depotwirkung.

Nach einem Ausgestaltungsmerkmal besteht das Protein zum überwiegenden Anteil aus Arginin. Vorteilhafterweise beträgt der Argininanteil mehr als 60 Gewichts%. Das Protein kann als Protamin, Protaminbase, Protaminderivate oder -salze, vorzugsweise Protaminsulfat oder Protaminchlorid vorliegen. Die vorgenannten Verbindungen haben vorteilhafterweise keine antigenen Eigenschaften.

Die, vorteilhafterweise einzelstängig ausgebildete, Nukleinsäuresequenz ist ein Oligonukleotid oder ein Derivat davon. Das Oligonukleotid besteht vorzugsweise aus mindestens 5 Nukleotiden. Das Derivat kann ein Phosphorothioat oder ein anionisches Derivat sein. Beim Oligonukleotid kann es sich insbesondere um ein DNA-Oligonukleotid handeln. Das ermöglicht einen Einsatz der synthetischen Partikel zur Antisense-Therapie.

Je nach Anwendungszweck kann der mittlere Durchmesser des Partikels im Bereich von 10 nm bis 100 µm liegen.

Das Partikel trägt vorteilhafterweise eine elektrische Oberflächenladung, die vorzugsweise im Bereich von -40 mV bis +40 mV liegen kann. Dadurch kann weiter die Transfektionseffizienz erhöht werden.

Nach der verfahrensseitigen Maßgabe der Erfindung ist ein Verfahren zur Herstellung der erfindungsgemäßen synthetischen Partikel mit folgenden Schritten vorgesehen:
a) Herstellen einer ein Protamin, eine Protaminbase, ein Protaminderivat oder -salz mit einem Molekulargewicht im Bereich von 3900 bis 4300 enthaltenden wässrigen ersten Lösung,
b) Versetzen der ersten Lösung mit einer ein Oligonukleotid oder einem Derivat davon enthaltenden zweiten Lösung, wobei das Gewichtsverhältnis zwischen Protamin und Oligonukleotid zwischen 1:1 und 5:1 beträgt und
c) Mischen der ersten und zweiten Lösung.

Das Verfahren ermöglicht auf einfache Weise die Herstellung der erfindungsgemäßen synthetischen Partikel.

Nach einem Ausgestaltungsmerkmal sind die erste und die zweite Lösung salzfrei. Zur Erzeugung einer vorgegebenen Oberflächenladung kann das molare Verhältnis von Nukleinsäure- oder Nukleinsäurederivatsequenz zu Protein eingestellt werden. Die vorgeschlagene Variante läßt sich besonders einfach durchführen.

Das Protein besteht zweckmäßigerweise zum überwiegenden Anteil aus Arginin, wobei es aus der folgenden Gruppe ausgewählt ist : Protamin, Protaminbase, Protaminderivate oder -salze, vorzugsweise Protaminsulfat oder Protaminchlorid. Insbesondere Protamin, Protaminbase oder Protaminderivate können aus dem Sperma von Lachs gewonnen werden. Eine einfache und billige Verfügbarkeit ist damit gewährleistet.

Die, vorteilhafterweise einzelstängig ausgebildete, Nukleinsäuresequenz ist ein Oligonukleotid oder ein Derivat davon. Das Oligonukleotid besteht vorzugsweise aus mindestens 5 Nukleotiden. Das Derivat kann ein Phosphorothioat oder ein anionisches Derivat sein. Der Durchmesser des Partikels kann je nach Anwendungszweck im Bereich von 10 nm bis 100 µm liegen. Es kann eine elektrische Oberfächenladung tragen, die zweckmäßigerweise im Bereich von -40 mV bis +40 mV liegt.

Nach einer weiteren Lösung der Aufgabe ist die Verwendung des oben genannten Proteins mit einem Molekulargewicht im Bereich von 3900 bis 4300 zur Herstellung eines ein Oligonucleotid bzw. Oligonukleotidderivat enthaltenden synthetischen Partikels vorgesehen.

Beim Oligonukleotid handelt es sich zweckmäßigerweise um ein DNA-Oligonukleotid.

Das erfindungsgemäße synthetische Partikel ist vorteilhafterweise ausschließlich aus einem Oligonukleotid oder einem Oligonukleotidderivat und dem oben genannten Protein mit dem Molekulargewicht im Bereich von 3900 bis 4300 gebildet. Das Molekulargewicht des Proteins beträgt nach einer besonders vorteilhaften Ausführungsform zwischen 4000 und 4250.

Nachfolgend werden Ausführungsbeispiele der Erfindung an Hand der Zeichnung und an Hand von Beispielen näher erläutert. In der Zeichnungen zeigen:
- Fig. 1a: eine rasterelektronenmikroskopische Aufnahme synthetischer Partikel mit negativer Oberflächenladung,
- Fig. 1b: eine rasterelektronenmikroskopische Aufnahme synthetischer Partikel mit positiver Oberflächenladung,
- Fig. 2: die Abhängigkeit der Partikelgröße von der Inkubationszeit,
- Fig. 3: die Abhängigkeit der Oberflächenladung vom Verhältnis Protamin/ Oligonukleotid,
- Fig. 4a: eine confokale laserscannmikroskopische Aufnahme einer ersten Vero-Zelle.
- Fig. 4b: eine confokale laserscannmikroskopische Aufnahme einer zweiten Vero-Zelle.
- Fig. 5: die Abhängigkeit der UV-Absorption bei 260 nm von der Retentionszeit für Partikel mit unterschliedleicher Protamin/Oligonukleotid-Zusammensetzung.

### Beispiele:

### 1. Die Herstellung von Oligonukleotidpartikel mit negativer Oberflächenladung

500 µl einer Protaminlösung (50 µg/ml) in doppelt destilliertem Wasser werden in einem Eppendorf-Cap bei Raumtemperatur spontan zu 500 µl einer ebenso salzfreien Oligonukleotidlösung (100 ug/ml) gegeben. Als Oligonukleotide sind vorzugsweise einzelsträngige DNA-Oligonukleotide in der Lösung enthalten. Die Lösung wird danach intensiv für 1 Minute mit einem schnell laufenden Rührwerk gemischt. Die Partikelbildung setzt dabei spontan ein und ist nach einer halben Stunde abgeschlossen. Das Gewichtsverhältnis zwischen dem eingesetzten Protaminmolekül und dem Oligonukleotid beträgt ca. 0,75 bis
1. Das Gewichtsverhältnis zwischen Protamin und dem Oligonukleotid zur Partikelbildung beträgt ca. 1:2,5.
2. Die Herstellung von Oligonukleotidpartikel mit positiver Oberflächenladung

Basierend auf dem Verfahren in Beispiel 1 beschriebenen Verfahren werden 500 µl einer Protaminlösung (250 µg/ml) in doppelt destilliertem Wasser in einem Eppendorf-Cap bei Raumtemperatur spontan zu 500 µl einer ebenso salzfreien Oligonukleotidlösung (100 µg/ml) gegeben. Das molare Verhältnis zwischen Protamin und dem Oligonukleotid beträgt ca. 3:1.

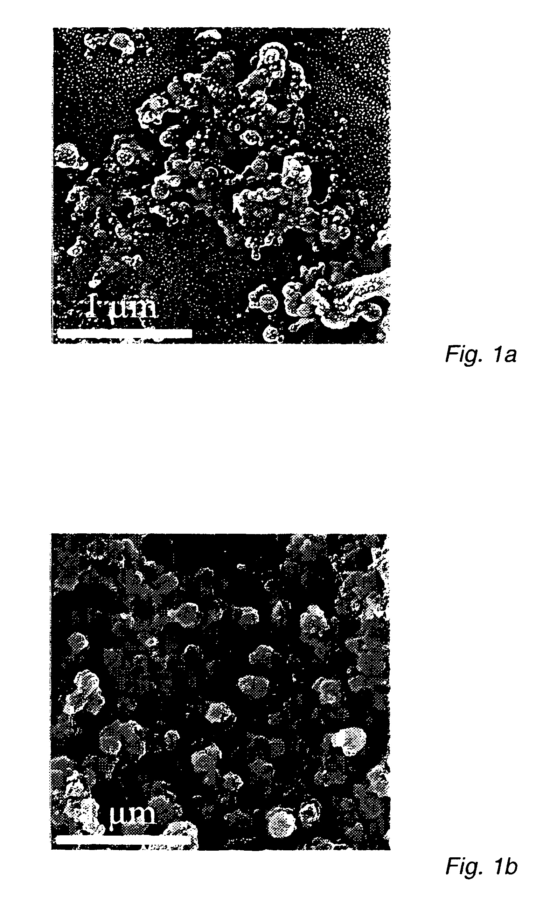

Fig. 1a zeigt eine rasterelektronenmikroskopische Aufnahme eines synthetischen Partikels mit negativer Oberflächenladung. Das Gewichtsverhältnis zwischen Protamin und Oligonukleotid hat hier 1:2 betragen. In Fig. 1b ist ein synthetisches Partikel mit positiver Oberflächenladung gezeigt. Das Gewichtsverhältnis zwischen Protamin und Oligonukleotid hat hier 2,5:1 betragen.

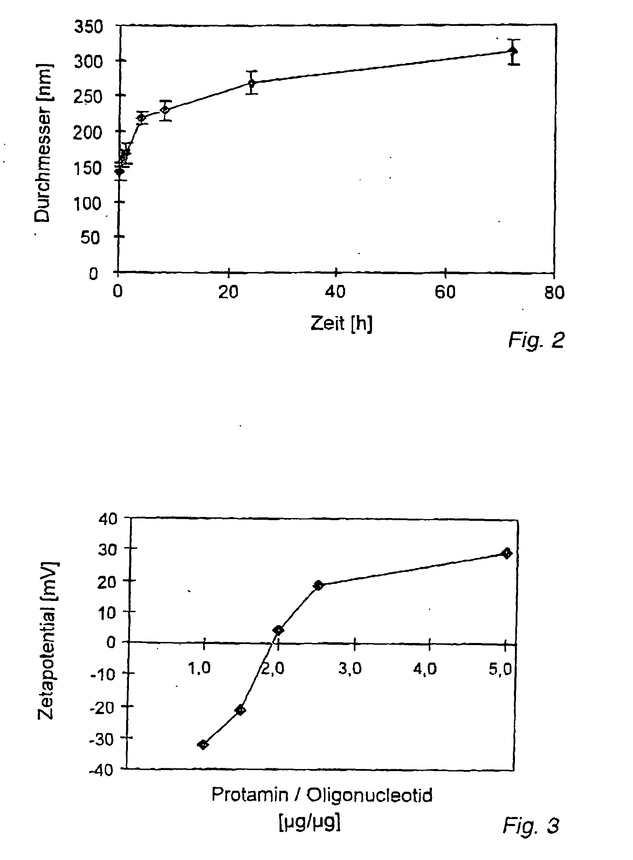

Fig. 2 zeigt die Abhängigkeit der Inkubationszeit vom Gewichtsverhältnis Protamin/Oligonukleotid. Mit zunehmender Inkubationszeit nimmt die Partikelgröße zu. Es können so beliebige Partikelgrößen eingestellt werden.

Fig. 3 zeigt die Abhängigkeit des Zetapotentials vom Gewichtsverhältnis Protamin/Oligonukleotid. Mit zunehmendem Anteil an Protamin verschiebt sich das Zetapotential zu positiven Werten hin.

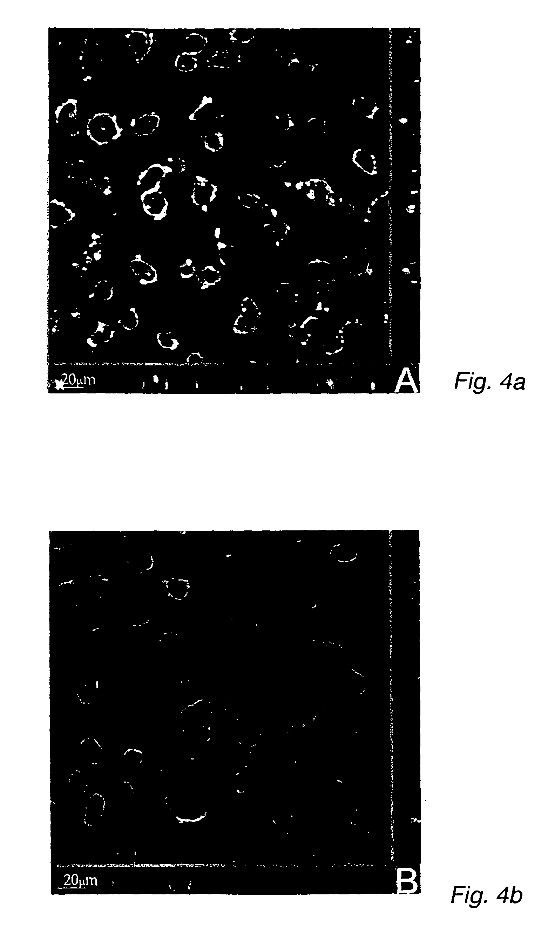

Die Fig. 4a und b zeigen im Vergleich die Aufnahme von Oligonukleotiden mittels synthetischer Partikel (Fig. 4a) in Vero-Zellen. In Fig. 4b ist eine Kontrollinkubation gelöster Oligonukleotide gezeigt. Die Oligonukleotidkonzentration beträgt 5 µg/ml bei einer Inkubationszeit von vier Stunden bei 37°C und 5% CO₂. Es zeigt sich, dass unter Verwendung der erfindungsgemäßen synthetischen Partikel Oligonukleotide verstärkt in Zellen aufgenommen werden.

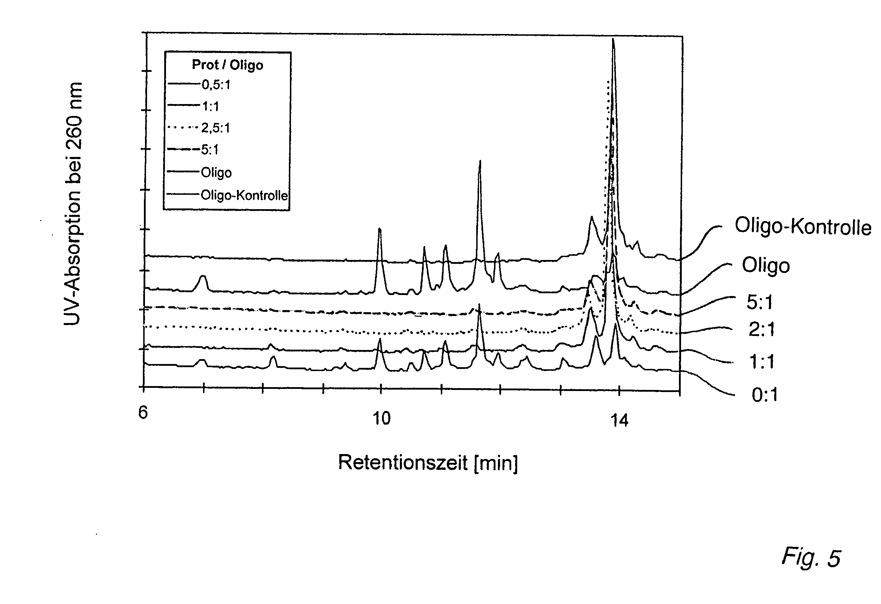

In Fig. 5 ist die Stabilität der erfindungsgemäßen Partikel gegenüber einem enzymatischen Abbau durch Endonukleasen gezeigt. Es ist die UV- Absorption bei 260 nm über der Retentionszzeit für verschiedene Protamin/Oigonukleotid-Verhältnisse aufgetragen. Die Ergebnisse zeigen, daß das erfindungsgemäße Partikel einen nahezu quantitativen Schutz vor einem enzymatischen Abbau gewährleistet.

## Patentansprüche

1. Synthetisches Partikel bestehend aus einem Oligonukleotid oder einem Derivat davon und einem Protamin, einer Protaminbase, einem Protaminderivat oder -salz mit einem Molekulargewicht im Bereich von 3900 bis 4300, wobei das Gewichtsverhältnis zwischen Protamin und Oligonukleotid zwischen 1:1 und 5:1 beträgt.

2. Synthetisches Partikel nach einem der vorhergehenden Ansprüche, wobei das Protaminsalz Protaminsulfat oder Protaminchlorid ist.

3. Synthetisches Partikel nach einem der vorhergehenden Ansprüche, wobei das Oligonukleotid einzelsträngig ausgebildet ist.

4. Synthetisches Partikel nach einem der vorhergehenden Ansprüche, wobei das Oligonukleotid aus mindestens 5 Nukleotiden besteht.

5. Synthetisches Partikel nach einem der vorhergehenden Ansprüche, wobei das Derivat ein Phosphorothioat oder ein anionisches Derivat ist.

6. Synthetisches Partikel nach einem der vorhergehenden Ansprüche, wobei der mittlere Durchmesser des Partikels im Bereich von 10 nm bis 100 µm liegt.

7. Synthetisches Partikel nach einem der vorhergehenden Ansprüche, wobei das Partikel eine elektrische Oberfächenladung trägt.

8. Synthetisches Partikel nach einem der vorhergehenden Ansprüche, wobei die Oberflächenladung im Bereich von -40 mV bis +40 mV liegt.

9. Verfahren zur Herstellung synthetischer Partikel nach einem der vorhergehenden Ansprüche mit folgenden Schritten:
a) Herstellen einer ein Protamin, eine Protaminbase, ein Protaminderivat oder -salz mit einem Molekulargewicht im Bereich von 3900 bis 4300 enthaltenden wässerigen ersten Lösung,
b) Versetzen der ersten Lösung mit einer ein Oligonukleotid oder ein Derivat davon enthaltenden zweiten Lösung, wobei das Gewichtsverhältnis zwischen Protamin und Oligonukleotid zwischen 1:1 und 5:1 beträgt und
c) Mischen der ersten und der zweiten Lösung.

10. Verfahren nach Anspruch 9, wobei die erste und die zweite Lösung salzfrei sind.

11. Verfahren nach Anspruch 9 oder 10, wobei zur Herstellung einer vorgegebenen Oberflächenladung das molare Verhältnis von Oligonukleotid oder dem Derivat davon zu Protein eingestellt wird.

12. Verfahren nach einem der Ansprüche 9 bis 11, wobei das Protaminsalz Protaminsulfat oder Protaminchlorid ist.

13. Verfahren nach einem der Ansprüche 9 bis 12, wobei Protamin, Protaminbase, Protaminderivate aus dem Sperma von Lachs gewonnen wird.

14. Verfahren nach einem der Ansprüche 9 bis 13, wobei das Oligonukleotid oder das Derivat davon einzelsträngig ausgebildet ist.

15. Verfahren nach einem der Ansprüche 9 bis 14, wobei das Oligonukleotid aus mindestens 5 Nukleotiden besteht

16. Verfahren nach einem der Ansprüche 9 bis 15, wobei das Derivat ein Phosphorothioat oder ein anionisches Derivat ist.

17. Verfahren nach einem der Ansprüche 9 bis 16, wobei der Durchmesser des Partikels im Bereich von 10 nm bis 100 µm liegt.

18. Verfahren nach einem der Ansprüche 9 bis 17, wobei das Partikel eine elektrische Oberfächenladung trägt.

19. Verfahren nach einem der Ansprüche 9 bis 18, wobei die Oberflächenladung im Bereich von -40mV bis +40 mV liegt.

20. Verwendung eines Protamins, einer Protaminbase, eines Protaminderivats oder -salzes mit einem Molekulargewicht im Bereich von 3900 bis 4300 zur Herstellung eines daraus und mindestens einem Oligonukleotid oder Derivat davon bestehenden synthetischen Partikels, wobei das Gewichtsverhältnis zwischen Protamin und Oligonukleotid zwischen 1:1 und 5:1 beträgt.

21. Verwendung nach Anspruch 20, wobei das Protaminsalz Protaminsulfat oder Protaminchlorid ist.

22. Verwendung nach einem der Ansprüche 20 oder 21, wobei das Oligonukleotid ein einzelsträngiges und/oder aus mindestens 5 Nukleotiden bestehendes Oligonukleotid oder ein als Phosphorothioat vorliegendes Derivat davon ist.

## Claims

1. Synthetic particle consisting of an oligonucleotide or a derivative thereto and a protamine, a protamine base, a protamine derivative or salt with a molecular weight in the range of 3900 to 4300, wherein the weight ratio between protamine and oligonucleotide is between 1:1 and 5:1.

2. Synthetic particle as defined in one of the preceding claims, wherein the protamine salt is protamine sulfate or protamine chloride.

3. Synthetic particle as defined in one of the preceding claims, wherein the oligonucleotide is single-stranded.

4. Synthetic particle as defined in one of the preceding claims, wherein the oligonucleotide consists of at least 5 nucleotides.

5. Synthetic particle as defined in one of the preceding claims, wherein the derivative is a phosphorothioate or an anionic derivative.

6. Synthetic particle as defined in one of the preceding claims, wherein the mean diameter of the particle is in the range from 10 nm to 100 µm.

7. Synthetic particle as defined in one of the preceding claims, wherein the particle carries an electrical surface charge.

8. Synthetic particle as defined in one of the preceding claims, wherein the surface charge is in the range from -40 mV to +40 mV.

9. Method for the manufacturing of synthetic particles as defined in one of the preceding claims with the following steps:
a) Manufacturing of a diluted first solution containing a protamine, a protamine base, a protamine derivative or salt with a molecular weight in the range from 3900 to 4300,
b) Adding to the first solution a second solution containing an oligonucleotide or a derivative thereto, wherein the weight ration between protamine and oligonucleotide is between 1:1 and 5:1 and
c) Mixing the first and the second solution.

10. Method as defined in claim 9, wherein the first and the second solution are salt-free.

11. Method as defined in claim 9 or 10, wherein the molar ratio of oligonucleotide or the derivative thereto to protein is set for the manufacturing of a specified surface charge.

12. Method as defined in one of the claims 9 to 11, wherein the protamine salt is protamine sulfate or protamine chloride.

13. Method as defined in one of the claims 9 to 12, wherein protamine, protamine base, protamine derivative is extracted from the sperm of salmon.

14. Method as defined in one of the claims 9 to 13, wherein the oligonucleotide or the derivative thereto is single-stranded.

15. Method as defined in one of the claims 9 to 14, wherein the oligonucleotide consists of at least 5 nucleotides.

16. Method as defined in one of the claims 9 to 15, wherein the derivative is a phosphorothioate or an anionic derivative.

17. Method as defined in one of the claims 9 to 16, wherein the diameter of the particle is in the range from 10 nm to 100 µm.

18. Method as defined in one of the claims 9 to 17, wherein the particle carries an electrical surface charge.

19. Method as defined in one of the claims 9 to 18, wherein the surface charge is in the range from -40 mV to +40 mV.

20. Use of a protamine, a protamine base, a protamine derivative or salt with a molecular weight in the range from 3900 to 4300 to manufacture a synthetic particle consisting of this and at least one oligonucleotide or derivative thereto, wherein the weight ratio between protamine and oligonucleotide is between 1:1 and 5:1.

21. Use as defined in claim 20, wherein the protamine salt is protamine sulfate or protamine chloride.

22. Use as defined in one of the claims 20 or 21, wherein the oligonucleotide is a single-stranded and/or an oligonucleotide consisting of at least 5 nucleotides or a derivative thereto available as phosphorothioate.

## Revendications

1. Particule synthétique comprenant un oligonucléotide ou un de ses dérivés et une protamine, une base de protamine, un dérivé ou sel de protamine d'une masse moléculaire comprise entre 3900 et 4300, le rapport pondéral entre protamine et oligonucléotide étant entre 1:1 et 5:1.

2. Particule synthétique selon l'une des revendications précédentes, le sel de protamine étant du sulfate de protamine ou du chlorure de protamine.

3. Particule synthétique selon l'une des revendications précédentes, l'oligonucléotide étant sous forme de simple brin.

4. Particule synthétique selon l'une des revendications précédentes, l'oligonucléotide comprenant au moins 5 nucléotides.

5. Particule synthétique selon l'une des revendications précédentes, le dérivé étant un phosphorothioate ou un dérivé anionique.

6. Particule synthétique selon l'une des revendications précédentes, le diamètre moyen de la particule étant compris entre 10 nm et 100 µm.

7. Particule synthétique selon l'une des revendications précédentes, la particule portant une charge de surface électrique.

8. Particule synthétique selon l'une des revendications précédentes, la charge de surface étant comprise entre -40 mV et +40 mV.

9. Procédé pour la fabrication de particules synthétiques selon l'une des revendications précédentes selon les opérations suivantes :
a) fabrication d'une première solution aqueuse comprenant une protamine, une base de protamine, un dérivé ou sel de protamine d'une masse moléculaire comprise entre 3900 et 4300,
b) addition la première solution avec une deuxième solution contenant un oligonucléotide ou un de ses dérivés, le rapport pondéral entre protamine et oligonucléotide étant compris entre 1:1 et 5:1 et
c) mélange de la première et de la deuxième solution.

10. Procédé selon la revendication 9, la première et la deuxième solution étant sans sel.

11. Procédé selon la revendication 9 ou 10, le rapport molaire d'oligonucléotide ou de son dérivé étant défini par rapport à la protéine pour la fabrication d'une charge de surface spécifiée.

12. Procédé selon l'une des revendications 9 à 11, le sel de protamine étant du sulfate de protamine ou du chlorure de protamine.

13. Procédé selon l'une des revendications 9 à 12, la protamine, la base de protamine, les dérivés de protamine étant obtenus du sperme de saumon.

14. Procédé selon l'une des revendications 9 à 13, l'oligonucléotide ou son dérivé étant sous forme de simple brin.

15. Procédé selon l'une des revendications 9 à 14, l'oligonucléotide comprenant au moins 5 nucléotides.

16. Procédé selon l'une des revendications 9 à 15, le dérivé étant un phosphorothioate ou un dérivé anionique.

17. Procédé selon l'une des revendications 9 à 16, le diamètre de la particule étant compris entre 10 nm et 100 µm.

18. Procédé selon l'une des revendications 9 à 17, la particule portant une charge de surface électrique.

19. Procédé selon l'une des revendications 9 à 18, la charge de surface étant comprise entre -40 mV et +40 mV.

20. Utilisation d'une protamine, d'une base de protamine, d'un dérivé ou d'un sel de protamine d'une masse moléculaire comprise entre 3900 et 4300 pour la fabrication d'une particule synthétique comprenant au moins un oligonucléotide ou son dérivé, le rapport pondéral entre protamine et oligonucléotide étant entre 1:1 et 5:1.

21. Utilisation selon la revendication 20, le sel de protamine étant du sulfate de protamine ou du chlorure de protamine.

22. Utilisation selon l'une des revendications 20 ou 21, l'oligonucléotide étant un oligonucléotide simple brin et/ou comprenant au moins 5 nucléotides ou un dérivé étant en structure phosphorothioate.
